Europäisches Patentamt

(19) European Patent Office

Office européen des brevets

(11) Numéro de publication: **0 231 163 B1**

(12) **FASCICULE DE BREVET EUROPEEN**

(45) Date de publication du fascicule du brevet:
18.04.90

(51) Int. Cl.⁴: **C07C 1/24**

(21) Numéro de dépôt: **87870013.7**

(22) Date de dépôt: **29.01.87**

(54) **Procédé d'obtention d'éthylène à partir d'éthanol.**

(30) Priorité: **30.01.86 LU 86284**

(43) Date de publication de la demande:
**05.08.87 Bulletin 87/32**

(45) Mention de la délivrance du brevet:
**18.04.90 Bulletin 90/16**

(84) Etats contractants désignés:
**AT BE DE ES FR GB IT NL SE**

(56) Documents cités:
**EP-A- 0 175 399**
**US-A- 4 049 573**
**US-A- 4 062 905**
**US-A- 4 148 835**
**US-A- 4 547 616**

**Le dossier contient des informations techniques
présentées postérieurement au dépôt de la demande et
ne figurant pas dans le présent fascicule.**

(73) Titulaire: **De Belgische Staat L'Etat Belge représenté
par le Secrétaire général des Services de
Programmation, de la politique scientifique Rue de la
Science, 8, B-1040 Bruxelles(BE)**

(72) Inventeur: **Jacobs, Julia, Sint-Jozeflei 24, B-2140 Malle
(West)(BE)**
Inventeur: **Tastenhoye, Paul, Eizerstraat 63,
B-1982 Tervueren(BE)**
Inventeur: **Jacobs, Pierre, Strijlandstraat 104,
B-1686 Goolk(BE)**
Inventeur: **Uytterhoeven, Jan B., Rotspoelstraat 361,
B-3030 Leuven (Heverlee)(BE)**

(74) Mandataire: **De Palmenaer, Roger et al, Bureau Vander
Haeghen 63, avenue de la Toison d'Or,
B-1060 Bruxelles(BE)**

## Description

La présente invention est relative à un procédé d'obtention d'éthylène à partir d'éthanol anhydre ou aqueux à l'aide d'un catalyseur du type zéolite cristallin d'aluminosilicate d'origine naturelle ou synthétique.

L'électroéquivalence de l'aluminium dans la structure cristalline des zéolites est équilibrée par la présence de cations dans la position anionique du réseau cristallin. Le cation est choisi parmi les éléments des groupes Ia, Ib, IIa, IIb, IIIa, IIIb, IVa, IVb, Va, VIb, VIIb et VIII du tableau de Mendeljev. Comme exemples de cations, on peut citer les métaux alcalins, les métaux alcalino-terreux et les lanthanides. Ces éléments doivent être d'une grosseur physique et d'une configuration appropriée pour diffuser dans les passages intracristallins de la structure du zéolite.

On connaît par le brevet US-A 4 062 905 un procédé catalytique pour convertir une charge constituée de méthanol, de diméthyl éther ou de leurs mélanges en des produits hydrocarbonés riches en éthylène et propylène. Le catalyseur utilisé dans ce procédé est un zéolite aluminosilicate cristallin présentant des pores de dimensions inférieures à 6 Å. Les zéolites qui peuvent être naturels ou synthétiques sont caractérisés par des cycles d'atomes d'oxygène à 8 éléments et présentent un rapport atomique Si/Al quelconque.

Ce brevet US-A 4 062 905 n'enseigne pas que les zéolites aluminosilicates cristallins présentant des pores formés par des cycles d'atomes d'oxygène à 8 éléments permettent une conversion d'environ 100% de l'éthanol avec une sélectivité d'environ 100% en carbone éthylénique.

Ce brevet US-A 4 062 905 n'enseigne également pas que des zéolites particuliers, les zéolites aluminosilicates cristallins présentant des pores formés par des cycles d'atomes d'oxygène à 8 et/ou 10 éléments et un rapport atomique Si/Al inférieur à environ 20, présentent une grande stabilité thermique.

On connaît également par le brevet US-A 4 148 835 un procédé pour convertir une charge d'alcool monohydrique en $C_1$-$C_4$, au moyen de catalyseurs tels que des zéolites aluminosilicates en produit hydrocarboné. Ce procédé présente une haute sélectivité pour la production d'oléfines, en particulier d'éthylène. Les zéolites utilisés dans ce procédé ont des pores de dimensions supérieures à 5 Å et sont, par exemple formés par des cycles d'atomes d'oxygène à 10 éléments. Les zéolites ont de préférence un rapport Si/Al supérieur à 24.

On connaît encore par le brevet US-A 4 547 616 un procédé de conversion catalytique de produits oxygénés tels que méthanol ou diméthyl éther en oléfines telles qu'éthylène, composés en $C_{3+}$. Les catalyseurs MTO (methanol to olefins) ont, de préférence, un rapport Si/Al d'au moins 24 et un indice de contrainte compris entre 1 et 12.

Ces brevets américains ne permettent pas de déduire les caractéristiques que doivent présenter un catalyseur pour obtenir un taux de transformation de l'éthanol d'environ 100% et une sélectivité en carbone éthylénique d'environ 100%, ce catalyseur présentant de plus une grande stabilité thermique.

De plus, le brevet US-A 4 062 905 enseigne que pour accroître la sélectivité en éthylène, il faut utiliser des catalyseurs présentant un rapport atomique Si/Al élevé.

Il est également connu, notamment par la demande de brevet européen n° 85 201 301.0, publiée sous le numéro 0 175 399, d'utiliser des catalyseurs du type aluminosilicate zéolitique cristallins contenant un silicate de métal $M_1$ ayant une valence égale à 3 et une coordination tétraédrique et contenant éventuellement un autre métal $M_2$ compensateur de charge, ces catalyseurs ayant une teneur en métal $M_1$ telle que le rapport molaire

$$\frac{M_1 - M_2/n}{Si + M_1}$$

(où n est la valence dudit autre métal) en pourcents est au maximum d'environ 1,5 , pour obtenir de l'éthylène pratiquement pur comme seul hydrocarbure à partir d'éthanol pur ou de solutions aqueuses même très diluées d'éthanol et ceci avec un taux de transformation de l'éthanol voisin de 100 %.

La présente invention est basée sur la constatation qu'il existe une classe de zéolites, comprenant de nombreux composés dont certains existent à l'état naturel, qui dans des conditions convenables, permettent de réaliser la conversion précitée d'éthanol en éthylène non seulement avec des rendements intéressants, mais encore avec une sélectivité en carbone éthylénique d'environ 100 % en poids.

La structure des zéolites peut être définie comme un réseau tridimensionnel de tétraèdres de forme $SiO_4$ et $AlO_4$ ayant chacun de leurs atomes d'oxygène en commun avec un autre tétraèdre. Selon la règle de Löwenstein, la restriction suivante existe en ce qui concerne l'arrangement des tétraèdres de $SiO_4$ et $AlO_4$ entre eux : deux tétraèdres de $AlO_4$ ne peuvent pas avoir en commun un ou plusieurs de leurs atomes d'oxygène, ou dans un réseau zéolitique deux tétraèdres $AlO_4$ ne peuvent pas être voisins l'un de l'autre.

EP 0 231 163 B1

Ce réseau, dont la géométrie varie d'un zéolite à l'autre, définit un système de cavités et/ou de pores dont les dimensions sont variables d'un zéolite à l'autre, mais qui sont uniformes pour un zéolite donné. Ces cavités sont reliées entre elles par des fenêtres, par des pores ou par d'autres cavités. Les pores sont soit parallèles et par conséquent donnent lieu à une porosité monodimensionnelle, soit s'intersectent dans deux ou dans les trois dimensions et forment dans ces cas une porosité bi- ou tridimensionnelle. Ces cavités sont occupées par des ions ou des molécules d'eau ayant une grande liberté de mouvement. Lesdits canaux ou pores sont formés par des cycles d'atomes d'oxygène à 8, 10 et/ou 12 éléments ou membres avec des ouvertures de dimensions variables exprimées en Angström (Å).

Les zéolites peuvent être différenciés les uns des autres par leur composition chimique et, plus spécialement et de façon précise, par leur spectre de diffraction des rayons X.

Des modifications mineures, quant aux espacements réticulaires et aux intensités observables sur certains diagrammes, sont dues à la quantité d'eau résiduelle dans les pores ou canaux, au remplacement de certains cations par d'autres ou à des variations du rapport silice/alumine, mais elles ne traduisent nullement une variation structurale du zéolite.

Une dimension déterminée des pores permet, pour un zéolite donné, d'absorber ou de rejeter des molécules déterminées.

On a découvert à présent que les catalyseurs d'une classe de zéolites définie par des paramètres précis non seulement permettent de transformer de l'öthanol anhydre ou aqueux en éthylène avec un taux de transformation d'environ 100% et une sélectivité en carbone éthylénique d'environ 100% en poids, mais présentent surtout une stabilité remarquable en fonction de leur temps d'utilisation.

Conformément à la présente invention, on utilise pour catalyser la transformation d'éthanol anhydre ou aqueux en éthylène au moins un catalyseur du type zéolite cristallin qui présente, d'une part, des canaux ou pores formés par cycles d'atomes d'oxygène à 8 et/ou 10 éléments ou membres et, d'autre part, un rapport atomique Si/Al inférieur à 20, ce catalyseur étant mis en contact avec de l'éthanol anhydre ou aqueux à une température comprise entre 400 et 800K (127 et 527°C) et à un débit de 0,05 à 10 heures⁻¹, de telle sorte qu'il présente une sélectivité en carbone éthylénique d'environ 100 % en poids et permette un taux de transformation d'environ 100 % de l'éthanol en éthylène.

On a constaté que les zéolites dont les ouvertures de pores sont formées par des cycles d'atomes d'oxygène à plus de 10 éléments, par exemple ceux dont les ouvertures de pores sont formées par des cycles d'atomes d'oxygène à 12 éléments ou membres, et/ou dont le rapport atomique Si/Al est supérieur à 20 présentent une médiocre stabilité en fonction du temps, même s'ils permettent d'obtenir un taux de transformation d'environ 100 % et présentent une sélectivité en carbone éthylénique d'environ 100% en poids, lorsqu'ils sont utilisés dans la réaction de déshydratation d'éthanol en éthylène.

Un grand nombre d'aluminosilicates zéolitiques cristallins présentent les deux paramètres caractéristiques définis plus haut conviennent pour être utilisés en tant que catalyseurs dans le cadre du procédé selon la présente invention.

Certains de ces catalyseurs, notamment ceux qui existent dans la nature, peuvent exiger un traitement après leur extraction, pour augmenter leur stabilité. Ainsi, ces catalyseurs naturels peuvent subir un échange ionique et/ou un traitement à haute température ou encore un traitement à l'aide d'un acide ou d'une base ou une combinaison de ces traitements.

Les aluminosilicates zéolitiques que l'on trouve dans la nature et qui présentent, après traitement éventuel, les deux paramètres précités sont, entre autres, les suivants : ferriérite, heulandite, clinoptilolite, stilbite, érionite, chabazite, lévinyte.

Selon une particularité de l'invention, on peut utiliser un mélange de zéolites naturels, dont l'un au moins a des pores ou canaux formés par des cycles d'atomes d'oxygène à 8 et/ou 10 éléments ou membres et présente un rapport atomique Si/Al inférieur à 20.

Comme zéolites synthétiques susceptibles d'être employés conformément à la présente invention, parce qu'ils présentent les deux paramètres critiques indiqués plus haut, on peut citer
- d'une part ceux qui correspondent à des zéolites naturels, par exemple ferriérite, chabazite, érionite,
- d'autre part ceux qui ne sont pas connus dans la nature, par exemple les zéolites ZSM-47 (ZSM-6), T, ZSM-22 et analogues.

Ces zéolites synthétiques font l'objet de brevets ou de publications.

Parmi les brevets concernant la préparation de zéolites synthétiques, on peut citer :
- le brevet US no 4.187.283 pour le zéolite ZSM-47 (ZSM-6)
- le brevet US no 2.950.952 pour le zéolite T
- le brevet européen no 0 102 716 pour le zéolite ZSM-22
- le brevet européen no 0 012 473 pour la ferriérite synthétique
- le brevet britannique no 574.911 pour la chabazite synthétique
- l'article de Hawkins, D.B. (Clays and Clay Minerals, vol. 29, p. 331-340 (1981)) pour la clinoptilolite.

Il est à noter que la caractérisation de la structure des zéolites non naturels, en particulier le zéolite ZSM-47 (ZSM-6) et le zéolite T, a été effectuée par hydroconversion du n-décane ( J.A. Martens, M. Tielen, P.A. Jacobs, J. Weitkamp, Zeolites 4, 98-107 (1984) ).

Comme cela est le cas dans la plupart des applications catalytiques des zéolites, il est préférable qu'au moins une partie des cations initialement présents dans le zéolite soit éliminée par échange ionique. Un tel échange permet de remplacer les cations par des ions hydrogène ou par des ions tels que, par exemple, les ions ammonium qui peuvent être dégradés en ions hydrogène par traitement thermique subséquent.

Le catalyseur peut être utilisé de diverses manières dans le procédé suivant l'invention. Ainsi, le catalyseur peut se présenter sous forme d'une couche fixe, d'un lit fluidisé ou de diverses couches de catalyseur transporté. Le catalyseur peut être utilisé avec ou sans liant et, le cas échéant, sous forme de produit extrudé.

Le procédé suivant la présente invention permet d'obtenir de l'éthylène sensiblement pur à partir d'éthanol anhydre ou d'éthanol aqueux, par exemple à partir d'alcool industriel titrant environ 94 % en poids d'éthanol ou d'un mélange de fermentation pouvant ne contenir qu'environ 4 % en poids d'éthanol.

L'éthanol anhydre ou aqueux est mis en contact avec le catalyseur, en phase gazeuse ou vapeur, éventuellement en présence d'un gaz inerte, tel que l'azote ou l'hélium, ce gaz pouvant être recyclé, si on le désire.

En aval de la couche de catalyseur, on obtient un mélange d'eau et de phase organique, cette dernière pouvant être aisément séparée de la phase aqueuse par simple refroidissement, de sorte que l'on récupère une phase organique composée presque exclusivement d'éthylène.

La liqueur éthanolique utilisée dans le procédé suivant l'invention peut provenir de la fermentation d'une biomasse ou d'un produit d'un gaz de synthèse dérivé de charbon ou de pétrole contenant de l'éthanol sous forme anhydre ou sous une forme plus ou moins concentrée avant de soumettre la liqueur éthanolique au procédé catalytique de transformation d'éthanol en éthylène suivant la présente invention. Le procédé permet ainsi de substantielles économies d'énergie, puisque la liqueur éthanolique de départ peut présenter une faible teneur en éthanol qui est presque intégralement transformé en éthylène grâce à la sélectivité remarquable des catalyseurs suivant la présente invention.

La température à laquelle est maintenu le catalyseur pour la transformation d'éthanol en éthylène peut varier entre des limites étendues, notamment entre 400 K et 800 K, de préférence entre environ 500 K et 700 K, cette température étant la température maximale atteinte dans la couche du catalyseur.

De même, le débit auquel on fait passer la liqueur éthanolique au contact du catalyseur peut également varier entre des limites étendues. Ainsi, ce débit, déterminé par la vitesse spatiale horaire pondérale (VSHP) qui correspond au poids d'éthanol amené en contact avec le catalyseur par heure et par poids de ce catalyseur, peut varier entre 0,05 et 10 heures$^{-1}$, de préférence entre 0,25 et 5,0 heures$^{-1}$.

Les valeurs optimales de la température du catalyseur et du débit de la liqueur éthanolique de départ peuvent être aisément déterminées par voie expérimentale, compte tenu de la teneur en éthanol de la liqueur de départ et du type particulier de catalyseur utilisé avec cette liqueur, en vue d'obtenir les résultats souhaités.

Le procédé suivant la présente invention peut être mis en oeuvre à des pressions de la liqueur éthanolique du départ et de l'éventuel gaz inerte servant de diluant comprises entre 1 et 10 atmosphères, mais on préfère opérer à la pression atmosphérique.

Les catalyseurs utilisés dans le procédé suivant l'invention ne subissent pas de dégénération et se caractérisent par une stabilité thermique et hydrothermique remarquablement élevée. Même en présence de quantités appréciables d'eau, les catalyseurs ne subissent pas de perte d'activité catalytique ou de sélectivité pendant une durée d'utilisation prolongée.

Les exemples suivants illustrent l'invention.

EXEMPLE 1 : Préparation de la forme hydrogène d'un zéolite ZEOLON 700 et utilisation de ce catalyseur pour la transformation d'éthanol en éthylène

(a) Préparation :

Le catalyseur a été préparé à partir d'un zéolite du commerce : ZEOLON 700 (Norton, lot n° 92017).

L'analyse par diffraction aux rayons X a révélé que le produit était constitué de ferriérite.

Pour la ferriérite, la composition élémentaire typique suivante est donnée par W.M. Meier et D.H. Olson ("Atlas of Zeolite Structure Types" published by the Structure Commission of the International Zeolite Association, printed by Juris Druck + Verlag AG, Zurich, Switzerland, 1978), p. 39 :
$Na_2 Mg_2 Al_6 Si_{30} O_{72} . 8 H_2O$.

Le zéolite ZEOLON 700 a été calciné à l'air à 823 K pendant 12 heures.

Puis le zéolite a été soumis à un échange ionique en le chauffant au reflux dans un excès de chlorure d'ammonium 0,5 N pendant 4 heures.

Après lavage et séchage, la poudre a été pressée, broyée et tamisée.

Ce zéolite traité présentait un système de canaux bidimensionnel, comportant des canaux formés par des cycles d'atomes d'oxygène à 10 éléments (4,3 x 5,5 Å) reliés à des canaux formés par des cycles d'atomes d'oxygène à 8 membres (3,4 x 4,8 Å). Ce zéolite avait un rapport atomique Si/Al de 5.

En vue d'être utilisé comme catalyseur, un échantillon du zéolite a été chargé dans un réacteur tubulaire à flux continu.

La forme hydrogène a été obtenue en calcinant le catalyseur dans le réacteur même à une température de 673 K sous un courant d'azote pendant 1 heure.

4

(b) Utilisation :

. Comme produit de départ, on a utilisé de l'éthanol aqueux de fermentation titrant environ 94 % en poids d'éthanol.

On a fait passer, en phase gazeuse, la solution aqueuse d'éthanol sur le catalyseur à une vitesse spatiale horaire pondérale en éthanol (VSHP) de 2,5 h$^{-1}$ conjointement avec 5 moles de gaz sec inerte comme diluant par mole d'éthanol. La durée de contact du réactif avec le catalyseur a été de 0,6 seconde.

La réaction a été exécutée à la pression atmosphérique.

Les produits de réaction ont été analysés en continu par chromatographie gazeuse en capillaire (voir tableau 1 plus loin).

EXEMPLE 2 : Préparation de la forme hydrogène d'un zéolite ZEOLON 400 et utilisation de ce catalyseur pour la transformation d'éthanol en éthylène

(a) Préparation :

Le catalyseur a été préparé à partir d'un zéolite du commerce : ZEOLON 400 (Norton, lot n° 43114)
L'analyse aux rayons X a prouvé que le produit était le zéolite clinoptilolite.
La composition élémentaire typique du zéolite clinoptilolite naturel est donnée par J.A. Breger et al (Am. Mineral., 55, 825 (1970) ) :
X$_2$O ; Al$_2$O$_3$ ; 10 SiO$_2$ ; 3 H$_2$O
où X est notamment le K et le Na, en moindre quantité le Ca et le Mg.
Le prétraitement a été effectué de la manière décrite dans l'exemple 1.
Ce zéolite présentait quatre types de canaux s'étendant dans trois directions différentes, à savoir des canaux à cycles de 10 éléments (7,05 x 4,25 Å), à cycles de 8 éléments (4,60 x 3,95 Å), à cycles de 8 éléments (5,40 x 3,90 Å) et à cycles de 8 éléments (5,20 x 3,90 Å). Le rapport atomique Si/Al était de 5.

(b) Utilisation :

On a opéré comme dans l'exemple 1. Les résultats de cet essai sont donnés dans le tableau 1.

EXEMPLE 3 : Préparation de la forme hydrogène d'un zéolite Chabazite et utilisation de ce catalyseur pour la transformation d'éthanol en éthylène

(a) Préparation :

Le catalyseur a été préparé à partir d'un zéolite naturel : la Chabazite de Bowie - Arizona.
L'identification du minéral a été faite par l'analyse aux rayons X.
La composition élémentaire typique pour la chabazite est donnée par W.M. Meier et D.H. Olson ("Atlas of Zeolite Structure Types", p. 25) :
Ca$_6$ Al$_{12}$ Si$_{24}$O$_{72}$ . 40 H$_2$O
Le zéolite a été traité de la manière décrite dans l'exemple 1, si ce n'est que l'on n'a pas calciné le zéolite à 823 K.
Ce zéolite se caractérisait par un système tridimensionnel de canaux formé par des cycles à 8 éléments ou membres (3,6 x 3,7 Å) et par un rapport atomique Si/Al de 2.

(b) Utilisation :

On a opéré dans les mêmes conditions réactionnelles que dans l'exemple 1. Les résultats obtenus sont donnés dans le tableau 1.

EXEMPLE 4 : Préparation de la forme hydrogène d'un zéolite Chabazite/Erionite et utilisation de ce catalyseur pour la transformation d'éthanol en éthylène

(a) Préparation :

Le catalyseur a été préparé à partir d'un zéolite naturel : la Chabazite/Erionite de Bowie, Arizona, U.S.A.
L'identification du minéral a été faite par l'analyse aux rayons X et a révélé la présence d'un mélange de chabazite et d'érionite ayant chacun des canaux tridimensionnels présentant des cycles à 8 éléments ou membres.
Le zéolite a été traité de la manière décrite dans l'exemple 3.
Le rapport Si/Al du zéolite était de 2-3.

(b) <u>Utilisation</u> :

On a opéré dans les mêmes conditions opérationnelles que dans l'exemple 1. Les résultats sont donnés dans le tableau 1.

<u>EXEMPLE 5</u> : Préparation de la forme hydrogène d'un zéolite Erionite et utilisation de ce catalyseur pour la transformation d'éthanol en éthylène

(a) <u>Préparation</u> :

Le catalyseur a été préparé à partir d'un zéolite naturel : érionite, Nevada, U.S.A.

L'analyse aux rayons X a prouvé que le produit était le minéral érionite ayant un système de canaux tridimensionnel, formé par des cycles à 8 membres (3,6 x 5,2 Å).

Pour l'érionite, la composition élémentaire typique suivante est donnée par W.H. Meier et D.H. Olson ("Atlas of Zeolite Structure Types", p. 35) :

$(Na_2, Ca...)_{4,5} Al_9 Si_{27} O_{72} . 27 H_2O$.

Le zéolite a été traité de la manière décrite dans l'exemple 3.

Le rapport Si/Al de ce zéolite était de 3.

(b) <u>Utilisation</u> :

On a opéré dans les mêmes conditions réactionnelles que dans l'exemple 1. Les résultats sont donnés dans le tableau 1.

Les résultats obtenus dans les exemples 1 à 5 sont consignés dans le tableau 1.

Ce tableau montre l'activité et la sélectivité en carbone éthylénique, dans la déshydratation d'éthanol en éthylène, pour les différents zéolites naturels.

Pour les résultats qui figurent dans ce tableau,

(1) la transformation théorique maximale d'éthanol en hydrocarbures et eau est toujours atteinte et est respectivement égale à 61 % et 39 % en poids

(2) le pourcentage de transformation est exprimé comme suit :

$$\frac{\text{quantité (en g) d'éthanol transformé}}{\text{quantité (en g) d'éthanol fourni}} \times 100$$

(3) la sélectivité en carbone éthylénique (% en poids) est exprimée comme suit :

$$\frac{\text{quantité (en g) d'éthylène formé}}{\text{quantité (en g) d'hydrocarbures formés}} \times 100$$

L'examen du tableau 1 montre que, dans tous les exemples, dans les conditions réactionnelles mentionnées, l'éthanol est totalement transformé et la sélectivité en carbone éthylénique est de 100 % en poids, sauf pour le H-ZEOLON 700 où elle est de 99,8 % en poids.

**Tableau 1: Catalyseurs zéolitiques naturels**

| Catalyseurs: | H-Zeolon 700 | H-Zeolon 400 | H-Chabazite | H-Chabazite/ Erionite | H-Erionite |
|---|---|---|---|---|---|
| Exemple no | 1 | 2 | 3 | 4 | 5 |
| Si/Al | 5 | 5 | 2 | 2–3 | 3 |
| Température (K) | 536 | 531 | 483 | 516 | 483 |
| VSHP ($h^{-1}$) | 2,5 | 2,5 | 2,5 | 2,5 | 2,5 |
| Temps de contact (sec) | 0,6 | 0,6 | 0,6 | 0,6 | 0,6 |
| Pression (atm) | 1,0 | 1,0 | 1,0 | 1,0 | 1,0 |
| Durée de l'essai: | | | | | |
| g éthanol/g catalyseur | 10,0 | 60,0 | 60,0 | 60 | 60 |
| Transformation (%) | 100,0 | 100,0 | 100,0 | 100,0 | 100,0 |
| Sélectivité pour $C_2H_4$ (% en poids) | 99,8 | 100,0 | 100,0 | 100,0 | 100,0 |

EXEMPLE 6 : Essai de durabilité du catalyseur H-ZEOLON 700 de l'exemple 1

Le catalyseur H-ZEOLON 700 a été préparé à partir du zéolite du commerce ZEOLON 700 (Norton) de la manière décrite dans l'exemple 1.

Un échantillon de ce catalyseur a été soumis à des traitements respectivement avec de l'acide chlorhydrique 0,5 N, avec de l'hydroxyde de sodium 0, 1 N et avec de l'acide chlorhydrique 0, 5 N chaque fois à une température de 353 K pendant une demi-heure.

Après chaque manipulation, le catalyseur a été lavé et séché à l'air. L'analyse aux rayons X a prouvé que la structure du zéolite était intacte après ce traitement. Ce zéolite avait la même structure que celui de l'exemple 1.

Les conditions réactionnelles et les résultats sont résumés dans le tableau 2.

Ce tableau montre que le catalyseur H-ZEOLON 700 présente d'abord un taux de conversion d'éthanol en éthylène de 100 % en même temps qu'une sélectivité en carbone éthylénique de 100 % en poids.

Le tableau montre également la désactivation rapide du catalyseur H-ZEOLON 700 non traité.

Par contre, on constate que les traitements à l'aide d'acide et d'une base augmentent considérablement la stabilité de ce même catalyseur pour la production sélective d'éthylène à partir d'éthanol aqueux (94 % en poids) obtenu par distillation d'une liqueur de fermentation.

Tableau 2

| Catalyseur: H-Zeolon 700 | non-traité | | | traité | | |
|---|---|---|---|---|---|---|
| **Conditions de réaction** | | | | | | |
| Température (K) | 536 | 536 | 536 | 536 | 536 | 536 |
| VSHP ($h^{-1}$) | 2,5 | 2,5 | 2,5 | 2,5 | 2,5 | 2,5 |
| Temps de contact (sec) | 0,6 | 0,6 | 0,6 | 0,6 | 0,6 | 0,6 |
| Pression (atm) | 1,0 | 1,0 | 1,0 | 1,0 | 1,0 | 1,0 |
| g éthanol/g catalyseur | 10 | 25 | 60 | 10 | 25 | 60 |
| Transformation (%) | 100,0 | 55,5 | 36,9 | 100,0 | 100,0 | 100,0 |
| **Distribution des produits (% en poids)** | | | | | | |
| Ether diéthylique | 0,0 | 15,0 | 6,0 | 0,0 | 0,0 | 0,0 |
| Acétaldehyde | 0,0 | 0,0 | 0,0 | 0,0 | 0,0 | 0,0 |
| Eau | 39,1 | 35,5 | 37,6 | 39,1 | 39,1 | 39,1 |
| Hydrocarbons | 60,9 | 49,5 | 56,4 | 60,9 | 60,9 | 60,9 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 100,0 | 100,0 | 100,0 | 100,0 | 100,0 | 100,0 |
| Ethane | 0,0 | 0,0 | 0,0 | 0,0 | 0,0 | 0,0 |
| Propylène + propane | 0,0 | 0,0 | 0,0 | 0,0 | 0,0 | 0,0 |
| $C_3^+$ | 0,0 | 0,0 | 0,0 | 0,0 | 0,0 | 0,0 |

EXEMPLE 7 : Préparation d'un catalyseur H-Ferriérite (Si/Al = 12) et transformation d'éthanol en éthylène à l'aide de ce catalyseur

(a) Préparation :

Ce catalyseur de type zéolite a été synthétisé par le procédé décrit dans l'exemple 9 du brevet EP 0 012 473.

La composition du mélange réactionnel, sur une base molaire, était la suivante :
93,5 $SiO_2$ / 4 $Al_2O_3$ / 10 $Na_2$ / 17 $Na_2SO_4$ / 36,7 pipéridine / 1938 $H_2O$ .

Le produit solide obtenu a été calciné à 823 K pendant 24 heures et analysé par diffraction aux rayons X. On a constaté qu'il s'agissait du zéolite ferriérite.

Le zéolite a été soumis à un échange ionique avec le cation ammonium en chauffant le catalyseur au reflux dans un excès de chlorure d'ammonium 0,5 N pendant 4 heures.

Ensuite, le catalyseur a été lavé l'eau et séché.

La forme hydrogène a été obtenue en calcinant le catalyseur dans le réacteur même à une température de 673 K sous un courant d'azote pendant 1 heure.

Sur base des résultats d'absorption atomique, le catalyseur cristallin obtenu présentait un rapport atomique Si/Al égal à 12. Par ailleurs, il présentait la même structure (canaux à cycles de 10 éléments et canaux à cycles de 8 éléments) que dans le zéolite de l'exemple 1.

(b) Utilisation :

Un échantillon de ce catalyseur a été chargé dans un réacteur tubulaire.

On a opéré de la même manière que dans l'exemple 1. Les résultats de cet essai sont donnés dans le tableau 3.

EXEMPLE 8 : Essai de durabilité du catalyseur H-Ferriérite de l'exemple 7

Un échantillon du catalyseur de l'exemple 7, chargé dans le réacteur tubulaire à flux continu et calciné à une température de 673 K, a été soumis à un essai de durée de vie.

Comme produit de départ, on a utilisé de l'éthanol aqueux provenant d'une liqueur de fermentation et titrant environ 94 % en poids d'éthanol.

On a fait passer cette solution sur le catalyseur en phase gazeuse.

Les conditions opératoires étaient les suivantes :
- vitesse spatiale horaire pondérale : 2,5 $h^{-1}$
- temps de contact : 0,6 seconde
- pression : atmosphérique
- température : 536 K
- durée de l'essai : jusqu'à 1000 g d'éthanol fourni par g de catalyseur.

On a constaté que l'éthanol a été quantitativement transformé en éthylène avec une sélectivité du catalyseur en carbone éthylénique de 99,8 % en poids.

Le taux de transformation d'éthanol en éthylène et la sélectivité en carbone éthylénique n'ont pas subi d'altération dans le temps.

Ce catalyseur H-Ferriérite présente donc une stabilité remarquable pour la production sélective d'éthylène à partir d'éthanol en présence d'eau.

EXEMPLE 9 : Préparation d'un catalyseur H-T et transformation d'éthanol en éthylène à l'aide de ce catalyseur

(a) Préparation :

On a préparé un zéolite du type T à partir des deux solutions suivantes :

solution 1 : 57,0 g de $SiO_2$ (aérosil) ;
18,3 g de NaOH ;
132,0 ml d'eau.
solution 2 : 10,0 g de $NaAlO_2$ ;
10,3 g de KOH ;
126,0 ml d'eau.

La solution 1 a été mélangée à la solution 2 en agitant de manière continue pendant 5 minutes.

La composition du mélange réactionnel, sur une base molaire, était la suivante :
20 $SiO_2$ $Al_2O_3$ 6 $Na_2O$ 310 $H_2O$ .

Le gel obtenu a été cristallisé dans des autoclaves pendant 7 jours à une température de 373 K sans agitation.

Le produit solide obtenu a été séparé de la solution liquide surnageante par filtration, puis lavé à l'eau et séché.

Un échange ionique avec le cation ammonium a été obtenu en chauffant le catalyseur au reflux dans un excès de chlorure d'ammonium 0,5 N, pendant 4 heures.

La forme hydrogène du catalyseur a été obtenue en calcinant le catalyseur dans le réacteur même à 623 K sous un courant d'azote pendant 1 heure.

On a ainsi obtenu un catalyseur ayant un rapport atomique Si/Al égal à 10.

La structure de ce zéolite analogue à celle de l'érionite et de l'offrétite a été caractérisée par hydroconversion de n-décane (Martens J.A., Tielen M., Jacobs P.A. et Weitkamp J., Zeolites, 4, 95-107, 1984) et a révélé un système de canaux tridimensionnel, formés de cycles à 8 membres.

(b) Utilisation :

Un échantillon de ce catalyseur a été chargé dans un réacteur tubulaire.

On a opéré de la même manière que dans l'exemple 1.

EXEMPLE 10 : Préparation d'un catalyseur H-ZSM-47 (ZSM-6) et transformation d'éthanol en éthylène à l'aide de ce catalyseur

(a) Préparation :

Le zéolite ZSM-47 (ZSM-6) a été préparé à partir des trois solutions suivantes :

solution 1 : 40,88 g de silice colloidale 40 % (Ludox AS-40, Du Pont, U.S.A.) ;
solution 2 : 6,15 g de chlorure de tétraméthylammonium (TMACl) ;
6,15 ml d'eau.
solution 3 : 1,967 g de $NaAlO_2$ ;
2,52 g de NaOH ;
13,5 ml d'eau.

Les trois solutions ont été mélangées en agitant vigoureusement pendant 15 minutes.

On a ainsi obtenu un gel ayant la composition molaire suivante :

28 $SiO_2$ $Al_2O_3$ 4 $Na_2O$ 6 TMACl 260 $H_2O$.

Ce gel a été cristallisé dans des autoclaves à une température de 453 K pendant 7 jours sans agitation, après quoi le produit a été lavé, séché à l'air et calciné à l'air à une température de 823 K pendant 12 heures.

Le zéolite obtenu a été ensuite soumis à un échange ionique avec le cation ammonium en chauffant le catalyseur au reflux dans un excès de chlorure d'ammonium 0,5 N pendant 4 heures.

Avant son utilisation comme catalyseur, le zéolite a été activé dans le réacteur même par calcination à 673 K sous un courant d'azote pendant 1 heure.

Ce catalyseur présentait un rapport atomique Si/Al de 15. Par hydroconversion de n-décane, on a constaté que ce zéolite était formé d'un système de canaux formés par des cycles d'atomes d'oxygène à 8 membres, comme pour l'érionite.

(b) <u>Utilisation</u> :

Un échantillon de ce catalyseur a été chargé dans un réacteur tubulaire.

On a opéré de la manière décrite dans l'exemple 1.

Dans le tableau 3 suivant figurent les résultats obtenus dans les exemples 7, 9 et 10.

Ce tableau montre, pour la réaction de déshydratation d'éthanol sur des zéolites synthétiques, les conditions opérationnelles, le taux de transformation et la sélectivité en carbone éthylénique.

Il ressort de ce tableau que dans les exemples 7, 9 et 10 et dans les conditions réactionnelles mentionnées, on obtient un taux de transformation de 100 % en même temps qu'une sélectivité en carbone éthylénique de 100 % en poids, sauf pour le Ferriérite où elle est de 99,8 % en poids.

Tableau 3: Catalyseurs zéolitiques synthétiques

| Catalyseurs: | H-Ferrierite | H-T | H-ZSM-47 |
|---|---|---|---|
| Exemple no | 7 | 9 | 10 |
| Si/Al | 12 | 10 | 15 |
| Température (K) | 536 | 512 | 625 |
| VSHP ($h^{-1}$) | 2,5 | 2,5 | 2,5 |
| Temps de contact (sec) | 0,6 | 0,6 | 0,6 |
| Pression (atm) | 1,0 | 1,0 | 1,0 |
| Durée de l'essai: | | | |
| g éthanol/g catalyseur | 1000 | 40 | 60 |
| Transformation (%) | 100,0 | 100,0 | 100,0 |
| Sélectivité pour $C_2H_4$ (% en poids) | 99,8 | 100,0 | 100,0 |

<u>EXEMPLE 11</u> : Préparation d'un catalyseur H-Ferriérite et transformation d'éthanol en éthylène à l'aide de ce catalyseur

(a) <u>Préparation</u> :

Ce catalyseur du type zéolite a été synthétisé par le procédé décrit dans l'exemple 8 du brevet EP 0.012.473.

La composition du mélange réactionnel, sur une base molaire, était la suivante :

93,5 $SiO_2$ 2 $Al_2O_3$ 17,7 $Na_2O$ 9,3 $Na_2SO_4$ 36,7 pipéridine 1938 $H_2O$.

Le produit solide a été calciné à 823 K pendant 24 heures et analysé par diffraction aux rayons X. On a constaté qu'il s'agissait du zéolite ferriérite ayant la même structure que celui de l'exemple 1.

La forme hydrogène du catalyseur a été obtenue de la manière décrite dans l'exemple 8.

Sur base des résultats d'absorption atomique, le catalyseur cristallin obtenu présentait un rapport atomique Si/Al égal à 23.

(b) <u>Utilisation</u> :

Un échantillon de ce catalyseur a été chargé dans un réacteur tubulaire.

On a opéré de la même manière que dans l'exemple 1.

Le tableau 4 suivant illustre les conditions réactionnelles et les résultats.

On a constaté, lors de ces essais, qu'il n'existe pas de conditions réactionnelles dans lesquelles le taux de transformation est de 100 % et la sélectivité en carbone éthylénique atteint plus de 99 % en poids.

Il convient de noter que le rapport atomique Si/Al est supérieur au maximum revendiqué : Si/Al = 20.

Ainsi, bien que ce zéolite ait une structure à canaux formés de cycles à 10 membres, liés à des canaux formés de cycles à 8 membres et répond ainsi au premier paramètre critique, il ne donne pas les résultats escomptés, étant donné que son second paramètre (rapport atomique Si/Al) est supérieur au maximum revendiqué.

Tableau 4

| Catalyseur: H-Ferrierite | | |
|---|---|---|
| Conditions de réaction | | |
| Température (K) | 490 | 520 |
| VSHP ($h^{-1}$) | 2,5 | 2,5 |
| Temps de contact (sec) | 0,6 | 0,6 |
| Pression (atm) | 1,0 | 1,0 |
| Transformation (%) | 86,3 | 100,0 |
| Distribution des produits (% en poids) | | |
| Ether diéthylique | 13,9 | 0,0 |
| Acétaldehyde | 0,0 | 0,0 |
| Eau | 35,7 | 39,1 |
| Hydrocarbures | 50,4 | 60,9 |
| CO | 0,0 | 0,0 |
| $CO_2$ | 0,0 | 0,0 |
| Distribution des hydrocarbures (% en poids) | | |
| Méthane | 0,0 | 0,0 |
| Ethylène | 99,4 | 78,9 |
| Ethane | 0,0 | 0,0 |
| Propylène + propane | 0,0 | 3,3 |
| $C_3^+$ | 0,6 | 17,8 |

EXEMPLE 12 : Préparation d'un catalyseur H-ZSM-22 et transformation d'éthanol en éthylène à l'aide de ce catalyseur

(a) Préparation :

On a préparé un zéolite du type ZSM-22 cristallin à partir des deux solutions suivantes :

solution 1 : 72 g de silice colloidale 40 % (Ludox AS-40, Du Pont, U.S.A.) ;
124 ml d'eau.
solution 2 : 3,54 g de $Al_2(SO_4)_3.18 H_2O$ ;
7,75 g de KOH ;
16,7 g de 1,6-diaminohexane ;
177 ml d'eau.

La solution 1 a été ajoutée à la solution 2 en agitant vigoureusement.

La composition du gel obtenu, sur base molaire, était la suivante :
90 $SiO_2$ $Al_2O_3$ 13 $K_2O$ 27 1,6-diaminohexane 3620 $H_2O$ .

Le mélange a été chauffé dans des autoclaves à 433 K pendant 2 jours, tout en agitant.

Après lavage, séchage et calcination à l'air à une température de 823 K pendant 24 heures, le produit solide a été analysé par diffraction aux rayons X. L'analyse a révélé la présence d'une phase fortement cristalline de ZSM-22.

Afin d'obtenir la forme hydrogène, le zéolite a été ensuite soumis à un échange ionique à 353 K à l'aide d'acide chlorhydrique 0,5 N en utilisant un rapport liquide/solide de 50 et en agitant de manière continue pendant une heure.

Avant son utilisation comme catalyseur, le zéolite a été activé dans le réacteur même par calcination à 673 K sous un courant d'azote pendant 1 heure.

Le catalyseur cristallin présentait un rapport atomique Si/Al de 45 sur base des résultats des analyses d'absorption atomique, tandis que sa structure se caractérisait par un système de canaux linéaire unidirectionnel et monodimensionnel formé par des cycles à 10 membres ayant des ouvertures de 5,5 x 4,5 Å. (Kokotailo G.T., Schlenker J.L., Dwyer F.G., Valyocsik E.W., Zeolites, 5, 349-351, 1985).

(b)Utilisation :

On a opéré de la même manière que dans l'exemple 1.

Les conditions opératoires et les résultats figurent au tableau 5.

Ces essais ont montré qu'il est impossible d'atteindre un taux de transformation quantitatif d'éthanol en même temps qu'une sélectivité en carbone éthylénique lorsqu'on utilise ce zéolite H-ZSM-22 présentant un rapport atomique Si/Al de 45, soit un rapport supérieur au maximum revendiqué.

### Tableau 5

| Catalyseur: H-ZSM-22 | | |
|---|---|---|
| **Conditions de réaction** | | |
| Température (K) | 512 | 526 |
| VSHP ($h^{-1}$) | 2,5 | 2,5 |
| Temps de contact (sec) | 0,6 | 0,6 |
| Pression (atm) | 1,0 | 1,0 |
| Transformation (%) | 97,2 | 100,0 |
| **Distribution des produits (% en poids)** | | |
| Ether diéthylique | 0,4 | 0,0 |
| Acétaldehyde | 0,0 | 0,0 |
| Eau | 39,0 | 39,1 |
| Hydrocarbures | 60,6 | 60,9 |
| CO | 0,0 | 0,0 |
| $CO_2$ | 0,0 | 0,0 |
| **Distribution des hydrocarbures (% en poids)** | | |
| Méthane | 0,0 | 0,0 |
| Ethylène | 99,7 | 96,5 |
| Ethane | 0,0 | 0,0 |
| Propylène + propane | 0,0 | 0,2 |
| $C_3^+$ | 0,3 | 3,3 |

EXEMPLE 13 : Préparation de la forme hydrogène d'un zéolite L et utilisation de ce catalyseur pour la transformation d'éthanol en éthylène

(a) Préparation :

Le catalyseur a été préparé à partir d'un zéolite du commerce : Linde type SK-45 (Union Carbide, U.S.A., lot n° 12508 - 86).

La composition élémentaire typique pour le zéolite Linde type L est donnée par W.M. Meier et D.H. Olson ("Atlas of Zeolite Structure Types", p. 59) :

$K_6 Na_3 Al_9 Si_{27} O_{27} . 21 H_2O$ .

Le zéolite a été soumis à un échange ionique avec le cation ammonium.

L'échange ionique a été effectué, à cinq reprises, en chauffant le catalyseur au reflux dans un excès de chlorure d'ammonium 0,5 N pendant 4 heures.

Après lavage et séchage, la poudre obtenue a été pressée, broyée et tamisée.

En vue d'être utilisé comme catalyseur, un échantillon du zéolite a été chargé dans un réacteur tubulaire à flux continu.

La forme hydrogène a été obtenue en calcinant le catalyseur dans le réacteur même à une température de 673 K sous un courant d'azote pendant 1 heure.

(b) Utilisation :

On a opéré de la manière décrite dans l'exemple 1. Le tableau 6 montre les conditions opératoires et les résultats obtenus.

Le zéolite H-L est capable de catalyser sélectivement la réaction de déshydratation d'éthanol en éthylène de façon à obtenir un taux de transformation quantitatif en même temps qu'une sélectivité en carbone éthylénique de 100 % en poids à une température de 508 K dans les conditions réactionnelles mentionnées.

Le tableau 6 montre cependant la désactivation (instabilité) rapide du catalyseur H-L : après la fourniture de 25 g d'éthanol par g de catalyseur, la transformation a déjà diminué de plus de 10 %.

Bien que le rapport atomique Si/Al de ce zéolite soit de 3, sa structure se caractérise par un système de canaux monodimensionnel formé de cycles à 12 membres (7,1 Å). Ce catalyseur ne répond donc pas aux deux paramètres critiques selon la présente invention.

### Tableau 6

| Catalyseur: H-L | | | | |
|---|---|---|---|---|
| Conditions de réaction | | | | |
| Température (K) | 508 | 508 | 508 | 508 |
| VSHP ($h^{-1}$) | 2,5 | 2,5 | 2,5 | 2,5 |
| Temps de contact (sec) | 0,6 | 0,6 | 0,6 | 0,6 |
| Pression (atm) | 1,0 | 1,0 | 1,0 | 1,0 |
| g éthanol/g catalyseur | 5 | 15 | 25 | 30 |
| Transformation (%) | 100,0 | 97,9 | 89,9 | 85,1 |
| Distribution des produits (% en poids) | | | | |
| Ether diéthylique | 0,0 | 1,3 | 6,1 | 8,7 |
| Acétaldehyde | 0,0 | 0,0 | 0,0 | 0,0 |
| Eau | 39,1 | 38,8 | 37,6 | 37,0 |
| Hydrocarbures | 60,9 | 59,9 | 56,3 | 54,3 |
| CO | 0,0 | 0,0 | 0,0 | 0,0 |
| $CO_2$ | 0,0 | 0,0 | 0,0 | 0,0 |
| Distribution des hydrocarbures (% en poids) | | | | |
| Méthane | 0,0 | 0,0 | 0,0 | 0,0 |
| Ethylène | 100,0 | 100,0 | 100,0 | 100,0 |
| Ethane | 0,0 | 0,0 | 0,0 | 0,0 |
| Propylène + propane | 0,0 | 0,0 | 0,0 | 0,0 |
| $C_3^+$ | 0,0 | 0,0 | 0,0 | 0,0 |

EXEMPLE 14 : Utilisation d'un zéolite H-ZEOLON 100 pour la transformation d'éthanol en éthylène

Le catalyseur du commerce H-ZEOLON 100 (Norton, U.S.A., lot n° 82 113) a été utilisé.

L'analyse aux rayons X a prouvé que le zéolite était du mordénite.

Pour le mordénite, la composition élémentaire typique suivante est donnée par W.M. Meier et D.H. Olson ("Atlas of Zeolite Structure Types", p. 69) :

$Na_8 Al_8 Si_{40} O_{96} . 24 H_2O$ .

La poudre a été pressée, broyée et tamisée.

En vue d'être employé comme catalyseur, un échantillon du zéolite a été chargé dans un réacteur tubulaire à flux continu.

Le zéolite a été activé dans le réacteur même par un prétraitement à 673 K sous un courant d'azote pendant 1 heure.

On a opéré de la même manière que dans l'exemple 1.

Les conditions réactionnelles et les résultats sont résumés dans le tableau 7 suivant.

Le tableau 7 montre que ce catalyseur H-ZEOLON 100 est en mesure de catalyser sélectivement la réaction de déshydratation d'éthanol en éthylène de façon à obtenir un taux de transformation quantitatif en même temps qu'une sélectivité en carbone éthylénique de 100 % en poids à une température de 495 K dans les conditions réactionnelles mentionnées.

Le tableau montre cependant la désactivation rapide du catalyseur H-ZEOLON 100.

Il convient de noter que la structure du mordénite est essentiellement bidimensionnelle, les canaux principaux étant formés par des cycles à 12 éléments (6, 7 x 7,0 Å) interconnectés à des canaux formés par des cycles à 8 éléments (2,9 x 5,7 Å), dont le rapport atomique Si/Al est cependant de 5. Ce zéolite ne présente pas les deux paramètres critiques des catalyseurs choisis conformément à la présente invention.

Tableau 7

| Catalyseur: H-Zeolon 100 | | | | | | |
|---|---|---|---|---|---|---|
| Conditions de réaction | | | | | | |
| Température (K) | 495 | 495 | 495 | 495 | 495 | 495 |
| VSHP ($h^{-1}$) | 2,5 | 2,5 | 2,5 | 2,5 | 2,5 | 2,5 |
| Temps de contact (sec) | 0,6 | 0,6 | 0,6 | 0,6 | 0,6 | 0,6 |
| Pression (atm) | 1,0 | 1,0 | 1,0 | 1,0 | 1,0 | 1,0 |
| g éthanol/g catalyseur | 12 | 22 | 27 | 32 | 42 | 62 |
| Transformation (%) | 100,0 | 98,4 | 85,5 | 54,6 | 25,4 | 15,3 |
| Distribution des produits (% en poids) | | | | | | |
| Ether diéthylique | 0,0 | 1,4 | 3,9 | 3,8 | 2,8 | 2,8 |
| Acétaldehyde | 0,0 | 0,0 | 0,0 | 0,0 | 0,0 | 0,0 |
| Eau | 39,1 | 38,8 | 38,2 | 38,2 | 38,4 | 38,4 |
| Hydrocarbures | 60,9 | 59,8 | 57,9 | 58,0 | 58,8 | 58,8 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| Distribution des hydrocarbures (% en poids) | | | | | | |
| Méthane | 0,0 | 0,0 | 0,0 | 0,0 | 0,0 | 0,0 |
| Ethylène | 100,0 | 100,0 | 100,0 | 100,0 | 100,0 | 100,0 |
| Ethane | 0,0 | 0,0 | 0,0 | 0,0 | 0,0 | 0,0 |
| Propylène + propane | 0,0 | 0,0 | 0,0 | 0,0 | 0,0 | 0,0 |
| $C_3^+$ | 0,0 | 0,0 | 0,0 | 0,0 | 0,0 | 0,0 |

**Revendications**

1. Procédé d'obtention d'éthylène à partir d'éthanol anhydre ou aqueux à l'aide d'un catalyseur du type zéolite cristallin d'aluminosilicate d'origine naturelle ou synthétique, caractérisé en ce qu'on utilise au moins un catalyseur du type zéolite cristallin qui présente, d'une part, des canaux ou pores formés par des cycles d'atomes d'oxygène à 8 et/ou 10 éléments ou membres et, d'autre part, un rapport atomique Si/Al inférieur à 20, ce catalyseur étant mis en contact avec l'éthanol anhydre ou aqueux à une température comprise entre 400 et 800K (127 et 527°C) et à un débit de 0,05 à 10 heures$^{-1}$, de telle sorte qu'il présente une sélectivité en carbone éthylénique d'environ 100% en poids et permette un taux de transformation d'environ 100% de l'éthanol en éthylène.

2. Procédé suivant la revendication 1, caractérisé en ce qu'on utilise au moins un zéolite naturel choisi parmi les suivants : ferriérite, heulandite, clinoptilolite, stilbite, érionite, chabazite, lévinyte, ainsi que leurs mélanges.

3. Procédé suivant la revendication 1, caractérisé en ce qu'on utilise au moins un zéolite synthétique choisi parmi les zéolites ZSM-47 (ZSM-6), T, ZSM-22 et analogues, ainsi que les ferriérite, chabazite, érionite et clinoptilolite synthétiques.

4. Procédé suivant l'une quelconque des revendications précédentes, caractérisé en ce qu'on utilise un catalyseur zéolitique soumis au préalable à au moins un des traitements suivants : un échange ionique et/ou un traitement à haute température ou encore un traitement à l'aide d'un acide ou d'une base ou une combinaison de ces traitements.

5. Procédé suivant l'une quelconque des revendications précédentes, caractérisé en ce qu'on utilise le catalyseur sous forme hydrogène.

6. Procédé suivant l'une quelconque des revendications précédentes, caractérisé en ce qu'on utilise une solution aqueuse d'éthanol contenant au moins 4 % en poids d'éthanol.

7. Procédé suivant la revendication 6, caractérisé en ce qu'on utilise une solution éthanolique constituée par une liqueur de fermentation d'une biomasse ou d'un produit d'un gaz de synthèse dérivé de charbon ou de pétrole contenant de l'éthanol.

## Patentansprüche

1. Verfahren zur Herstellung von Ethylen aus wasserfreiem oder wasserhaltigem Ethanol mit Hilfe eines Katalysators in der Art eines Zeolithen aus natürlich vorkommendem oder synthetischem kristallinem Aluminosilicat, dadurch gekennzeichnet, daß man zumindest einen Katalysator in Form eines kristallinen Zeolithen verwendet, der einerseits durch 8- und/oder 10-gliedrige Sauerstoffringe Kanäle oder Poren aufweist und andererseits das Atomverhältnis Si:Al <20 ist, und ihn mit dem wasserfreien oder wasserhaltigen Ethanol bei einer Temperatur von 400 bis 800K (127 bis 527°C) und einer Durchsatzgeschwindigkeit von 0,05 bis $10h^{-1}$ in Berührung bringt, so daß sich eine Selektivität an ethylenischem Kohlenstoff von etwa 100 Gew.-% ergibt und der Umsetzungsgrad von Ethanol zu Ethylen etwa 100% ist.

2. Verfahren nach Anspruch 1, dadurch gekennzeichnet, daß man einen natürlich vorkommenden Zeolith in Form von Ferrierit, Heulandit, Clinoptilolit, Stilbit, Erionit, Chabasit, Levinyt oder deren Gemische verwendet.

3. Verfahren nach Anspruch 1, dadurch gekennzeichnet, daß man zumindest einen synthetischen Zeolith in Form von Zeolithen der Bezeichnung ZSM-47 (ZSM-6), T, ZSM-22 und deren Analoge sowie auch synthetische Ferrierit, Chabasit, Erionit und Clinoptilolit verwendet.

4. Verfahren nach einem der vorhergehenden Ansprüche, dadurch gekennzeichnet, daß man einen zeolithischen Katalysator verwendet, der zumindest einer der folgenden Behandlungen unterzogen worden ist: Ionenaustausch und/oder Behandlung bei hoher Temperatur oder Behandlung mit einer Säure oder einer Base oder eine Kombination dieser Behandlungen.

5. Verfahren nach einem der vorhergehenden Ansprüche, dadurch gekennzeichnet, daß man den Katalysator in der Wasserstoffform verwendet.

6. Verfahren nach einem der vorhergehenden Ansprüche, dadurch gekennzeichnet, daß man eine wässrige Ethanollösung mit zumindest 4 Gew.-% Ethanol verwendet.

7. Verfahren nach Anspruch 6, dadurch gekennzeichnet, daß man eine Ethanollösung verwendet, der die Fermentationsflüßigkeit einer Biomasse oder ein Produkt von Synthesegas aus Kohle oder Erdöl mit einem Ethanolgehalt verwendet.

## Claims

1. Process for obtaining ethylene from anhydrous or aqueous ethanol by means of a catalyst of the crystalline aluminosilicate zeolite type of natural or synthetic origin, characterized in that at least one catalyst of the crystalline zeolite type is used, said catalyst having, on the one hand, channels or pores formed by cycles of oxygen atoms having 8 and/or 10 elements or members and, on the other hand, an atomic Si/Al ratio of less than 20, this catalyst being contacted with anhydrous or aqueous ethanol at a temperature comprised between 400 and 800K (127 and 527°C) and at a flow of 0.05 to 10 hours$^{-1}$, so that it has a carbon selectivity for ethylene of about 100% by weight and has a rate of conversion of about 100% of ethanol into ethylene.

2. Process according to claim 1, characterized in that at least one natural zeolite selected among the followings: ferrierite, heulandite, clinoptilolite, stilbite, erionite, chabazite, levinyte and mixtures thereof, is used.

3. Process according to claim 1, characterized in that at least one synthetic catalyst selected among the ZSM-47 (ZSM-6), T, ZSM-22 zeolites and the like, as well as synthetic ferrierite, chabazite, erionite and clinoptilolite, is used.

4. Process according to anyone of the preceding claims, characterized in that a zeolite catalyst previously submitted to at least one of the following treatments: an ion exchange and/or a treatment at a high temperature or even a treatment by means of an acid or a base or a combination of these treatments, is used.

5. Process according to anyone of the preceding claims, characterized in that the catalyst is used under the hydrogen form.

6. Process according to anyone of the preceding claims, characterized in that an aqueous solution of ethanol containing at least 4% by weight of ethanol is used.

7. Process according to claim 6, characterized in that an ethanol solution consisting of a liquor of fermentation of biomass or of a product of synthesis gas derived from coal or petroleum containing ethanol is used.